(19) Europäisches Patentamt

European Patent Office

Office européen des brevets

(11) EP 0 563 974 B1

(12) **EUROPÄISCHE PATENTSCHRIFT**

(45) Veröffentlichungstag und Bekanntmachung des Hinweises auf die Patenterteilung:
**19.11.1997 Patentblatt 1997/47**

(51) Int Cl.$^6$: **G01N 21/35, A61B 5/00**

(21) Anmeldenummer: **93105452.2**

(22) Anmeldetag: **01.04.1993**

(54) **Verfahren und Vorrichtung zum Messen des Partialdrucks verschiedener Komponenten eines Gasgemisches**

Method and device for measuring the partial pressure of different components of a gas mixture

Procédé et appareil pour mesurer la pression partielle des composants différents d'un mélange de gaz

(84) Benannte Vertragsstaaten:
**BE DE FR GB IT NL**

(30) Priorität: **01.04.1992 DE 4210829**

(43) Veröffentlichungstag der Anmeldung:
**06.10.1993 Patentblatt 1993/40**

(73) Patentinhaber: **Erich Jaeger GmbH**
**D-97204 Hoechberg (DE)**

(72) Erfinder:
• **Küchler, Gert, Dr.**
**W-8702 Kist (DE)**

• **Erdmann, Udo**
**W-8706 Hoechberg (DE)**

(74) Vertreter: **von Bezold, Dieter, Dr. et al**
**Dr. Dieter von Bezold**
**Dipl.-Ing. Peter Schütz**
**Dipl.-Ing. Wolfgang Heusler**
**Brienner Strasse 52**
**80333 München (DE)**

(56) Entgegenhaltungen:
**WO-A-90/04164 GB-A- 2 186 076**
**US-A- 4 226 692**

**Beschreibung**

Bei der Analyse von Gasgemischen müssen in Fällen, für die universelle Meßverfahren wie Massenspektroskopie und Gaschromatographie nicht geeignet sind, zur Bestimmung der Anteile der verschiedenen Gaskomponenten je nach Anforderungen unterschiedliche selektive oder begrenzt selektive Meßverfahren angewendet werden. So lassen sich mit der Infrarotabsorptionsspektroskopie nur die heteroatomigen Gase selektiv erfassen, die durch spezielle Absorptionswellenlängen charakterisiert sind, während die Messung von einatomigen Edelgasen und den zweiatomigen Elementgasen wie z.B. Sauerstoff mit diesem Meßprinzip nicht möglich ist und eine andere Methode erfordert. Andererseits ist beispielsweise zur Analyse von Oxidationsprozessen etwa in der Umweltmeßtechnik oder der industriellen Gasmeßtechnik und insbesondere auch zur medizinischen Gasanalyse die simultane Erfassung sowohl von Sauerstoff als auch seiner Reaktionsprodukte erforderlich, so daß zwei oder mehr selektiv arbeitende Meßverfahren eingesetzt werden müssen. Der Sauerstoffpartialdruck läßt sich vorteilhaft durch Festkörperionenleiter bestimmen, die sich durch geringe Kosten, gute Meßdynamik und gute Selektivität auszeichnen. Zur Bestimmung von Oxidationsprodukten wie beispielsweise $CO_2$, $CO$, $NO_2$ und $NO$ hat sich dagegen die IR-Absorptionsspektroskopie bewährt.

Die getrennte Messung von Gasanteilen mit unterschiedlichen Meßverfahren ist aber oft problematisch, etwa wenn zusätzlich Informationen über den zeitlichen Verlauf der Partialdruckänderungen in einem Gasgemisch benötigt werden, da sich besonders bei schnellen Partialdruckänderungen weder gleiche Meßbedingungen noch genaue zeitliche Zuordnung einhalten lassen.

Festkörperionenleiter können in den aus der Mikroelektronik bekannten Dick- oder Dünnschichttechniken od. dgl. als miniaturisierte Bauelemente hergestellt werden, etwa mit durch Siebdruck oder Sputtern auf einem flachen Keramiksubstrat mit einem Durchmesser von weniger als 1 cm aufgebrachten sensitiven Schichten (A.S. Ioannou, W. C. Maskell, KTV Gratton (Ed), Aden Hilger, Bristol, 1991; J. Gerblinger, H. Meixner, J. Appl. Phys. 67 (12), 1990, 7453-7459). Charakteristisch für solche Festkörperionenleiter ist, daß der dem Meßprinzip zugrunde liegende physikalische Effekt erst bei relativ hohen Temperaturen von mehr als 500°C praktisch meßbar ist. Auch die Dynamik des Ladungsträgertransports wird mit zunehmender Temperatur schneller, so daß zum Erreichen kleiner Ansprechzeiten im Interesse einer schnellen Messung hohe Betriebstemperaturen von mehr als 800°C angestrebt werden. Die erwähnten bekannten Sensoren sind daher an der Rückseite des Substrates mit Heizungen in Dickschicht- oder Dünnschichttechnik versehen.

Zur Vermeidung von Meßfehlern muß die erforderliche hohe Betriebstemperatur der Festkörperionenleiter möglichst konstant gehalten werden. Hierfür sind bisher verschiedene Möglichkeiten bekannt, die in der Praxis zu beträchtlichen Schwierigkeiten führen. Beispielsweise wurden Thermoelemente aus Draht oder in Dick- oder Dünnschichttechnik aufgebracht, deren Kontaktierungspunkte unter Umständen in der Gasströmung, also in einem Bereich undefinierter Temperatur plaziert sind, und/oder deren Referenzpunkt in zu geringer Entfernung vom beheizten, zu messenden Körper plaziert ist, so daß die Temperaturänderungen des Referenzpunktes direkt als Fehler in die Messung der Sensortemperatur eingeht. Eine andere Möglichkeit war das Aufbringen temperaturabhängiger Widerstände in ähnlicher Technik, die bei Temperaturen oberhalb 800°C zu Instabilität neigen. Generell leiten an der sensitiven Oberfläche des Festkörperionenleiters aufgebrachte Temperaturmeßelemente durch die Kontaktierung Energie ab, was eine inhomogene Temperaturverteilung an der Oberfläche und entsprechende Meßfehler zur Folge hat. Einen genauen Meßwert für die gesamte sensitive Fläche können diese Meßelemente nicht liefern. Außerdem wird durch die endliche Masse der aufgebrachten Temperaturmeßelemente insbesondere bei schnellen Änderungen der Meßbedingungen die Meßdynamik begrenzt, so daß keine zeitlich hochauflösende Gaskonzentrationsbestimmung im erwünschten Maße möglich ist.

Der Erfindung liegt die Aufgabe zugrunde, ein Verfahren bzw. eine Vorrichtung gemäß dem Oberbegriff der Ansprüche 1 bzw. 2 bzw. 9 anzugeben, die eine einfache und genaue Messung des Partialdrucks der Komponenten eines Gasgemisches ermöglichen.

Diese Aufgabe wird durch die kennzeichnenden Merkmale der Ansprüche gelöst.

Die Erfindung ermöglicht eine simultane Messung des Gaspartialdrucks verschiedener, nach nur einem einzigen Meßprinzip nicht erfaßbarer Gaskomponenten, nämlich einerseits ein- oder zweiatomiger Gase wie $O_2$ und andererseits heteroatomiger Gase, dennoch unter weitgehend gleichen Meßbedingungen und mit hoher zeitlicher Auflösung und praktisch vernachlässigbar geringer Verzögerung.

Gemäß einem ersten Aspekt der Erfindung wird die Absorption der Wärmestrahlung der sensitiven Oberfläche des Festkörperionenleiters durch ein oder mehrere heteroatomige Gase des Gasgemisches optoelektronisch gemessen. Durch das Ausnützen der sensitiven Oberfläche des beheizten Festkörperionenleiters als Strahlungsquelle für das IR-Absorptionsmeßverfahren lassen sich die beiden an sich unterschiedlichen Meßprinzipien simultan unter denselben Meßbedingungen wie Meßvolumen, Druck, Gasströmung und Gaskonzentration anwenden. Insbesondere sind strömungsbedingte zeitliche Verzögerungen zwischen den beiden Meßverfahren praktisch vernachlässigbar. Durch konstruktive Ausgestaltung wie Schichtdicke und Material der sensitiven Schicht des Festkörperionenleiters und geeignete Wahl der Temperatur läßt sich die Dynamik der beiden Meßverfahren aufeinander abstimmen, so daß sich z.B. gleiche Signalanstiegszeiten ergeben. Hohe zeitliche Auflösung der Gaskonzentrationsmessungen mit Anstiegszeiten von weniger als 20 ms sind für beide Meßverfahren möglich.

Das hier beschriebene Meßverfahren eignet sich besonders für die zeitlich hoch auflösende Messung der gasförmigen Reaktionsprodukte von Oxidationsvorgängen, namentlich in der medizinischen Diagnostik für die Analyse von Atemgasen ($O_2$, $CO_2$) innerhalb eines Atemzuges.

Gemäß einem anderen Aspekt der Erfindung wird die Wärmestrahlung der sensitiven Oberfläche des Festkörperionenleiters in einem ausgewählten Spektralbereich optoelektronisch gemessen und mit dem Strahlungsempfängersignal stabilisiert. Insbesondere kann die Heizung des Festkörperionenleiters von dem Strahlungsempfängersignal zur Temperaturregelung gesteuert werden. Dadurch werden die oben erwähnten Nachteile der bisher üblichen Methoden zur Temperaturkonstanthaltung vermieden und die Meßgenauigkeit der $O_2$-Bestimmung sowie ggf. der IR-Absorptionsmessung verbessert. Wenn die Wärmestrahlung zur Temperaturregelung im Wellenlängenbereich des ansteigenden Teils der Emissions-Wellenlängenfunktion des strahlenden Festkörperionenleiters gemessen wird, je nach Temperatur also im IR-Wellenlängenbereich unterhalb 2 µm, wird eine Verfälschung des Signals durch Absorption durch die heteroatomigen Gase vermieden. Zweckmäßig ist beispielsweise eine Messung im Bereich um etwa 1 µm.

Die genannte Kurve und die spektrale Zusammensetzung der Strahlung des Festkörperionenleiters entsprechen annähernd der bekannten Emissionsgleichung eines schwarzen Körpers

$$E(\alpha,T) = \frac{2\pi h \cdot c^2 \cdot \alpha^{-5}}{(e^{h \cdot c/(\alpha \cdot k \cdot T)} -1)}$$

mit

E ($\alpha$,T) - spektrale Emissionsenergie [$Wm^{-2}\mu m^{-1}$]
$\alpha$ - Wellenlänge [µm]
h - Planks Konstante 6,6256 . $10^{-34}$[Js]
k - Boltzmann Konstante 1,3805 . $10^{-23}$ [$JK^{-1}$]
c - Lichtgeschwindigkeit im Vakuum 2,998 . $10^8$ [m/s]

Das Maximum der Emissionswellenlängenfunktion ergibt sich unter Verwendung von Wiens Verschiebungsgesetz zu

$$\alpha_{max} \cdot T = \text{konstant} = 2897,6 \ \mu m \ K$$

Das bedeutet, für Temperaturen innerhalb des Bereichs von 500-1000°C wird 2,27619 µm < $\alpha_{max}$ < 3,7485 µm.

Die Wellenlänge der optoelektronisch gemessenen Wärmestrahlung kann durch ausgewählte spektrale Übertragungseigenschaften einer die Strahlung zum Strahlungsempfänger führenden optischen Faser und/oder des Strahlungsempfängers begrenzt werden.

Durch die Erfindung kann also die Wärmestrahlung eines Festkörperionenleiters ausgenutzt werden sowohl zur sinnvollen Kombination von zwei an sich verschiedenen Gasmeßverfahren, nämlich eines selektiven Verfahrens und eines Verfahrens mit eingeschränkter Selektivität ausgenutzt werden, deren Ergebnisse unter weitgehender Vermeidung von Meßfehlern gleichzeitig zur Verfügung stehen, als auch für eine zusätzliche Verbesserung der Meßgenauigkeit speziell durch Vermeiden von Temperaturfehlern. An einem bevorzugten Ausführungsbeispiel einer Vorrichtung zum Durchführen des beschriebenen Verfahrens wird die Erfindung im folgenden näher erläutert. In der Zeichnung zeigen:

Fig. 1    eine schematische Prinzipdarstellung der Vorrichtung;

Fig. 2    eine konstruktiv zweckmäßige Ausführungsform einer Meßkammer für die Vorrichtung nach Fig. 1; und

Fig. 3    eine Draufsicht auf eine Gasaustrittsdüse in der Meßkammer nach Fig. 2.

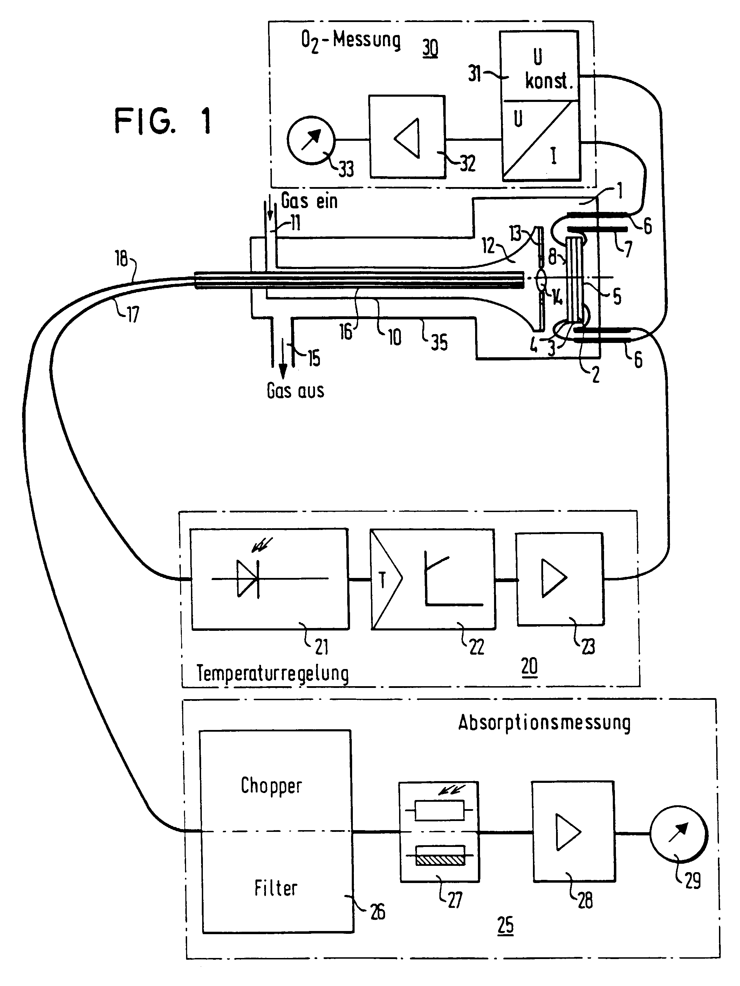

Gemäß Fig. 1 ist in einer Meßkammer 1 ein Festkörperionenleiter 2 montiert, der im wesentlichen aus einem scheibenförmigen Substrat 3 beispielsweise aus Keramik, einer auf der einen Substratseite befindlichen sensitiven Schichtstruktur 4 und einer auf der Rückseite des Substrates befindlichen mäanderförmigen Heizleiterbahn 5 besteht. Die Schichtstruktur 4 und die Heizleiterbahn 5 sind jeweils über dünne Drähte mit Anschlußstiften 6 bzw. 7 verbunden. Festkörperionenleiter dieser Art sind an sich in verschiedenen Ausführungsformen bekannt und nicht Gegenstand der Erfindung.

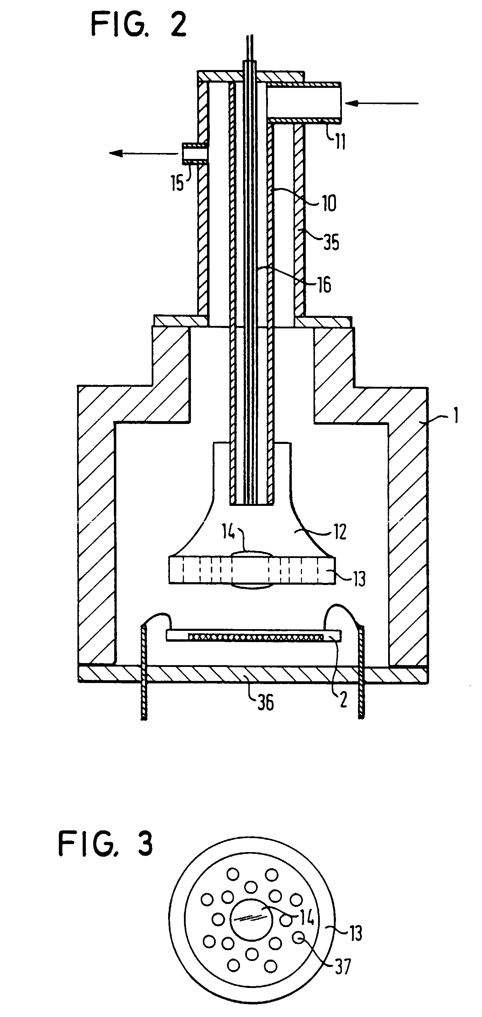

Koaxial zur Scheibenachse des Festkörperionenleiters 2 befindet sich in einem zylinderförmigen oder kolbenartigen Teil 35 der Meßkammer 1 auf der der sensitiven Oberfläche 8 der Schichtstruktur 4 zugewandten Seite ein Rohr 10, das an dem vom Festkörperionenleiter entfernten geschlossenen Ende einen mit einer externen Gasleitung verbundenen Gaseinlaß 11 hat und am anderen Ende in einen Düsenkopf 12 mündet, der bis auf eine Vielzahl von Gasaustrittsöffnungen (Fig. 3) in einer mit Abstand parallel zu der sensitiven Oberfläche 8 liegenden Gasaustrittsplatte 13 für das einströmende Gas geschlossen ist. Im Zentrum der Gasaustrittsplatte 13, also koaxial zur Scheibenachse des Festkörperionenleiters 2 ist eine optische bikonvexe Linse 14 eingebaut, die beispielsweise aus $CaFl_2$ bestehen kann.

Innerhalb des das Gas in den Düsenkopf 12 leitenden Rohres 10 befindet sich koaxial ein dünnes Metallrohr 16, das am axialen Ende des zylinder- oder kolbenförmigen Teils 35 der Meßkammer 1 aus dieser herausragt. In diesem Metallrohr 16 sind zwei verschiedene optische Fasern geführt, deren Strahlungsaufnahmeenden etwa am Brennpunkt der Linse 14 fixiert sind, nämlich beispielsweise eine HCS-Glasfaser 17 und eine Fluorid-Glasfaser 18 bekannter Art. Die numerische Apertur der Linse 14 ist an die kleinste numerische Apertur der Glasfasern 17,18 angepaßt.

Die HCS-Glasfaser 17 führt zu einem beispielsweise aus einer Si-Photodiode gebildeten Strahlungsempfänger 21 am Eingang eines Temperaturregelkreises 20. Entsprechend ihrer spektralen Übertragungseigenschaften mit einem Dämpfungsminimum bei 1,2 µm überträgt die Glasfaser 17 weitgehend nur den Anteil der Strahlung des Festkörperionenleiters im Bereich unterhalb $\alpha_{max}$ der eingangs erwähnten Kurve zu der Photodiode, deren spektrale Empfindlichkeitskennlinie durch ein Maximum bei ungefähr 1 µm charakterisiert sein kann. Somit steht also ein im ansteigenden Teil der Emissions-Wellenlängenfunktion liegender selektierter Teil der Strahlung des beheizten Ionenleiters zur Verfügung. Die Strahlungsabsorption durch das zwischen der Linse 14 und dem Festkörperionenleiter 2 befindliche Gasgemisch kann in diesem Wellenlängenbereich vernachlässigt werden.

Am Ausgang des Strahlungsempfängers 21 steht eine Spannung als Maß für die Temperatur der sensitiven Schichtstruktur 4 des Festkörperionenleiters zur Verfügung, die an den Eingang eines nachgeschalteten PI-Reglers 22 angelegt wird. Über eine Leistungsstufe 23 und die Anschlußstifte 7 steuert der Regler 22 die Heizleiterbahn 5 des Festkörperionenleiters 2 im Sinne einer Temperaturkonstanthaltung. Hierbei können Regelabweichungen von weniger als 0,15 K für eingestellte Solltemperaturen im Bereich von 800 bis 1000°C und eine Zeitkonstante der Regelung von 15 ms erreicht werden.

Die zweite optische Glasfaser 18, die als Fluoridglasfaser generell durch geringe Dämpfung im Infrarotwellenlängenbereich gekennzeichnet ist, führt dagegen in einen Absorptionsmeßkreis 25, an dessen Eingang sich eine aus einem Metallinterferenzfilter und einem Chopper gebildete Selektionseinheit 26 befindet, welche in an sich bekannter Weise selektiv nur die spektralen Anteile der Strahlung aus der Glasfaser 18 durchläßt, bei der eine Absorption durch das jeweils zu messende Gas erfolgt. Von der Selektionseinheit 26 gelangt die Strahlung zu einem beispielsweise aus einem PbSe-Bauelement gebildeten Strahlungsempfänger 27, dessen Signal über einen Verstärker 28 eine Anzeigeeinrichtung 29 steuert. Das bekannte Prinzip dieser Absorptionsmessung bedarf keiner näheren Erläuterung.

Zur $O_2$-Messung wird das Ausgangssignal des Festkörperionenleiters 2, der bei dem beschriebenen Beispiel in an sich bekannter Weise nach dem amperometrischen Meßprinzip arbeitet, also aufgrund seiner Leitfähigkeitsänderungen einen dem Partialdruck der betreffenden Gaskomponente entsprechenden Strom erzeugt, über die Anschlußstifte 6 einem Strom-Spannungswandler 31 am Eingang eines $O_2$-Meßkreises 30 zugeführt. Der Strom-Spannungswandler 31 steuert über einen Verstärker 32 eine Anzeigeeinrichtung 33.

Im Betrieb wird das zu messende Gasgemisch durch den Einlaß 11 angesaugt (beispielsweise mit einer Strömungsrate in der Größenordnung von 300 ml/min) und durch die Gasaustrittsplatte 13 des Düsenkopfes 12 gleichmäßig auf die gesamte sensitive Oberfläche des Festkörperionenleiters 2 verteilt.

Durch die beschriebene konstruktive Anordnung werden Konzentrationsänderungen im anströmenden Gas nahezu gleichzeitig an der gesamten sensitiven Oberfläche 8 wirksam, und strömungsbedingte Verzögerungen werden auf ein Minimum herabgesetzt. Durch die Linse 14, die relativ temperaturbeständig ist, wird die Wärmestrahlung der gesamten sensitiven Oberfläche gesammelt. Während sich das Gasgemisch im Zwischenraum zwischen der Gasaustrittsplatte 13 und der sensitiven Oberfläche 8 der Schichtstruktur 4 befindet und die $O_2$-Komponente den erwähnten Ausgangsstrom des Festkörperionenleiters 2 steuert, wird gleichzeitig vom Absorptionsmeßkreis 25 die Absorption der Wärmestrahlung durch eine oder mehrere heteroatomige Gaskomponenten des Gemisches gemessen. Von dem Zwischenraum zwischen der Gasaustrittsplatte 13 und der sensitiven Oberfläche wird das Gasgemisch dann außerhalb des Rohres 10 durch den zylinder- oder kolbenförmigen Teil des Gehäuses durch einen Gasauslaß 15 abgesaugt.

Durch die Glasfasern 17,18 wird eine vollkommene thermische Entkopplung der Strahlungsquelle, also des erhitzten Festkörperionenleiters von den jeweiligen Strahlungsempfängern im Temperaturregelkreis 20 und im Absorptionsmeßkreis 25 gewährleistet. Die Messung der Temperatur der sensitiven Oberfläche 8 des Festkörperionenleiters erfolgt ohne Rückwirkung auf die Temperaturverteilung dieser Oberfläche. Statt der bevorzugten räumlichen Trennung wären in Sonderfällen allerdings auch andere Möglichkeiten einer thermischen Entkopplung denkbar.

Die thermische Belastung der optischen Glasfasern 17,18 kann u.a. dadurch in den erforderlichen Grenzen gehalten werden, daß sie durch die Gasgemischströmung innerhalb des Rohres 10 gekühlt werden.

Es kann auch zweckmäßig sein, einen Wärmetauscher vorzusehen, in dem das von dem Festkörperionenleiter erwärmte, durch den Gasauslaß 15 abgesaugte Gasgemisch das der Meßkammer durch den Gaseinlaß 11 zugeführte Gemisch aufheizt. Beispielsweise geschieht dies bei der dargestellten Vorrichtung innerhalb der Meßkammer im Rohr 10.

In konstruktiver Hinsicht zeichnet sich die beschriebene Vorrichtung ferner vor allem durch ein minimales Meßvolumen, kompakte Bauweise und geringen Bauaufwand der Meßkammer aus.

In Fig. 2 ist eine mögliche Ausführungsform der Meßkammer 1 mit dem einen axialen Fortsatz der eigentlichen Kammer bildenden Kolbenteil 35, in dem sich der Gaseinlaß 11 und der Gasauslaß 15 befinden, dem an den Gaseinlaß 11 angeschlossenen Rohr 10, dem die Glasfasern enthaltenden inneren Metallrohr 16, dem auf das Rohr 10 aufgeschobenen Düsenkopf 12 mit seiner Gasaustrittsplatte 13 und der Linse 14 sowie mit dem hier nur schematisch gezeigten Festkörperionenleiter 2 dargestellt. Die Meßkammer kann im wesentlichen aus einem innen polierten Messinggehäuse bestehen, auf das ein Kolbenteil 35 mit geringerem Durchmesser aufgesetzt ist, und das an seinem entgegengesetzten unteren Ende einen Deckelteil 36 hat, der den Festkörperionenleiter 2 trägt. Darstellungsgemäß kann das Ende der Glasfaser in derselben Ebene liegen wie die unteren Enden der Rohre 10 und 16. Bei einem praktisch realisierbaren Beispiel kann die Absorptionslänge zwischen der sensitiven Oberfläche des Festkörperionenleiters 2 und dem Ende der Glasfaser, nämlich die Summe der beiden Abstände zwischen der Linse 14 und der sensitiven Oberfläche bzw. dem Ende der Glasfaser ungefähr 8 mm oder weniger betragen.

Fig. 3 zeigt eine Draufsicht auf die Gasaustrittsplatte 13 in der Meßkammer nach Fig. 2, aus der die zweckmässige Verteilung der Gasaustrittsöffnungen 37 erkennbar ist.

**Patentansprüche**

1. Verfahren zum Messen des Partialdrucks verschiedener Komponenten eines Gasgemisches, das über eine für ein bestimmtes Gas sensitive Oberfläche eines Festkörperionenleiters geleitet wird, der auf eine für die Messung dieses Gases erforderliche Temperatur aufgeheizt wird,
**dadurch gekennzeichnet**,
daß die Absorption der Wärmestrahlung der sensitiven Oberfläche des Festkörperionenleiters durch ein oder mehrere heteroatomige Gase des Gemisches optoelektronisch gemessen wird.

2. Verfahren zum Messen des Partialdrucks von Komponenten eines Gasgemisches, das über eine für ein bestimmtes Gas sensitive Oberfläche eines Festkörperionenleiters geleitet wird, der auf eine für die Messung dieses Gases erforderliche Temperatur aufgeheizt wird,
**dadurch gekennzeichnet**, daß die Wärmestrahlung der sensitiven Oberfläche des Festkörperionenleiters in einem ausgewählten Spektralbereich optoelektronisch gemessen und mit dem Strahlungsempfängersignal stabilisiert wird.

3. Verfahren nach Anspruch 2, **dadurch gekennzeichnet**, daß die Heizung des Festkörperionenleiters von dem Strahlungsempfängersignal zur Temperaturregelung gesteuert wird.

4. Verfahren nach Anspruch 2 oder 3, **dadurch ge-**

**kennzeichnet**, daß die Wärmestrahlung im Wellenlängenbereich des ansteigenden Teils der Emissions-Wellenlängenfunktion des strahlenden Festkörperionenleiters gemessen wird.

5. Verfahren nach Anspruch 4, **dadurch gekennzeichnet**, daß die Strahlung im IR-Wellenlängenbereich unterhalb 2 µm, insbesondere um etwa 1 um gemessen wird.

6. Verfahren nach einem der vorangehenden Ansprüche, **dadurch gekennzeichnet**, daß die Wellenlängen der optoelektronisch gemessenen Wärmestrahlung durch ausgewählte spektrale Übertragungseigenschaften einer die Strahlung zum Strahlungsempfänger führenden optischen Faser und/oder des Strahlungsempfängers begrenzt werden.

7. Verfahren nach Anspruch 6, **dadurch gekennzeichnet**, daß die optische Faser durch die Gasgemischströmung gekühlt wird.

8. Verfahren nach einem der vorangehenden Ansprüche, **dadurch gekennzeichnet**, daß das von dem Festkörperionenleiter erwärmte abgeführte Gasgemisch durch Wärmetausch das dem Festkörperionenleiter zugeführte Gasgemisch aufheizt.

9. Vorrichtung zum Messen des Partialdrucks verschiedener Komponenten eines Gasgemisches, das über eine für ein bestimmtes Gas sensitive Oberfläche (8) eines Festkörperionenleiters (2) geleitet wird, der auf eine für die Messung dieses Gases erforderliche Temperatur aufgeheizt wird, mit einer Meßkammer (1), die den mit einer Heizvorrichtung (5) versehenen Festkörperionenleiter (2) enthält und einen Gaseinlaß (11) sowie einen Gasauslaß (15) für das Gasgemisch hat,
**dadurch gekennzeichnet**,
daß in der Meßkammer (1) die Wärmestrahlung der sensitiven Oberfläche in eine Strahlungsempfangsanordnung (17,18) geleitet wird, wobei sich zwischen dem Ende der Strahlungsempfangsanordnung (17,18) und der sensitiven Oberfläche (8) das zu messende Gasgemisch befindet, und daß die Strahlungsempfangsanordnung (17,18) mit einem optoelektronischen Empfänger (27) eines Absorptionsmeßkreises (25) zum Messen der Absorption der Wärmestrahlung durch ein heteroatomiges Gas und/oder mit einem optoelektronischen Empfänger (21) eines die Heizvorrichtung (5) steuernden Temperaturregelkreises (20) verbunden ist.

10. Vorrichtung nach Anspruch 9, **dadurch gekennzeichnet**, daß die Strahlungsempfangsanordnung durch eine faseroptische Wellenleiteranordnung (17,18) gebildet ist, die mit ihrem Eingangsende in einem Abstand von der sensitiven Oberfläche (8) angeordnet ist und zu dem optoelektronischen Strahlungsempfänger (27) des Absorptionsmeßkreises (25) und/oder zu dem optoelektronischen Strahlungsempfänger (21) des Temperaturregelkreises (20) führt.

11. Vorrichtung nach Anspruch 9 oder 10, **dadurch gekennzeichnet**, daß das Gasgemisch in einer im wesentlichen senkrecht zu der sensitiven Oberfläche (8) des Festkörperionenleiters (2) liegenden Richtung gegen die sensitive Oberfläche (8) geleitet wird.

12. Vorrichtung nach Anspruch 11, **dadurch gekennzeichnet**, daß das Gasgemisch von dem Gaseinlaß (11) der Meßkammer (1) durch ein Rohr (10) in einen Düsenkopf (12) fließt, der eine im Abstand parallel zu der sensitiven Oberfläche liegende Gasaustrittsplatte (13) mit einer über die Fläche verteilten Vielzahl von Gasaustrittsöffnungen (37) hat.

13. Vorrichtung nach Anspruch 12, **dadurch gekennzeichnet**, daß das Gasleitungsrohr (10) und der Düsenkopf (12) konzentrisch oder achsparallel zu dem Eingangsende der Wellenleiteranordnung (17,18) liegen.

14. Vorrichtung nach Anspruch 13, **dadurch gekennzeichnet**, daß das Gasleitungsrohr (10) die Wellenleiteranordnung (17,18) umschließt und in der Mitte der Gasaustrittsplatte (13) des Düsenkopfes (12) eine optische Linse (14) angeordnet ist, die die Wärmestrahlung der sensitiven Oberfläche des Festkörperionenleiters (2) sammelt, und daß das Ende der Wellenleiteranordnung (17,18) im Bereich des Brennpunkts der Linse (14) liegt.

15. Vorrichtung nach einem der Ansprüche 10 bis 14, **dadurch gekennzeichnet**, daß die Wellenleiteranordnung aus zwei optisch getrennten Fasern (17,18) besteht, von denen die eine zu dem Absorptionsmeßkreis (25) und die andere zu dem Strahlungsempfänger (21) des Temperaturregelkreises (20) führt.

16. Vorrichtung nach Anspruch 15, **dadurch gekennzeichnet**, daß die zum Temperaturregelkreis (20) führende Glasfaser (17) ein spektrales Dämpfungsminimum bei ungefähr 1 um und der Strahlungsempfänger (21) des Temperaturregelkreises (20) eine maximale Empfindlichkeit bei ungefähr 1 um hat.

17. Vorrichtung nach einem der Ansprüche 10 bis 16, **dadurch gekennzeichnet**, daß am Eingang des Absorptionsmeßkreises (25) eine mit Chopper und Filter arbeitende Wellenlängenselektionseinheit (26) zwischen die ankommende Glasfaser (18) und den Strahlungsempfänger (27) geschaltet ist.

## Claims

1. Method of measuring the partial pressure of different components of a gas mixture which is conducted over a surface, which is sensitive to a predetermined gas, of a solid body ionic conductor, which is heated to a temperature necessary for the measurement of this gas, characterised in that the absorption of the thermal radiation from the sensitive surface of the solid body ionic conductor is opto-electronically measured by one or more heteroatomic gases of the mixture.

2. Method of measuring the partial pressure of components of a gas mixture which is conducted over a surface, which is sensitive to a predetermined gas, of a solid body ionic conductor, which is heated to a temperature necessary for the measurement of this gas, characterised in that the thermal radiation from the sensitive surface of the solid body ionic conductor is opto-electronically measured in a selected spectral region and is stabilised with the radiation receiver signal.

3. Method as claimed in Claim 2, characterised in that the heating of the solid body ionic conductor is controlled by the radiation receiver signal for the purpose of temperature control.

4. Method as claimed in Claim 2 or 3, characterised in that the thermal radiation is measured in the wavelength region of the rising portion of the emission wavelength function of the radiating solid body ionic conductor.

5. Method as claimed in Claim 4, characterised in that the radiation is measured in the IR wavelength region below 2 $\mu m$, particularly round about 1 $\mu m$.

6. Method as claimed in one of the preceding claims, characterised in that the wavelengths of the opto-electronically measured thermal radiation are limited by selected spectral transmission characteristics of an optical fibre conducting the radiation to the radiation receiver and/or of the radiation receiver.

7. Method as claimed in Claim 6, characterised in that the optical fibre is cooled by the gas mixture flow.

8. Method as claimed in one of the preceding claims, characterised in that the heated gas mixture conducted away from the solid body ionic conductor heats the gas mixture supplied to the solid body ionic conductor by heat exchange.

9. Apparatus for measuring the partial pressure of different components of a gas mixture which is conducted over a surface (8), which is sensitive to a predetermined gas, of a solid body ionic conductor (2), which is heated to a temperature necessary for the measurement of this gas, including a measuring chamber (1), which contains the solid body ionic conductor (2), provided with a heating device (5), and has a gas inlet (11) and a gas outlet (15) for the gas mixture, characterised in that in the measuring chamber (1) the thermal radiation from the sensitive surface is conducted into a radiation receiving system (17, 18), the gas mixture to be measured being situated between the end of the radiation receiving system (17, 18) and the sensitive surface (8), and that the radiation receiving system (17, 18) is connected to an opto-electronic receiver (27) of an absorption measuring circuit (25) for measuring the absorption of the thermal radiation by a heteroatomic gas and/or to an opto-electronic receiver (21) of a temperature control circuit (20) controlling the heating device (5).

10. Apparatus as claimed in Claim 9, characterised in that the radiation receiving system is constituted by a fibre optical wave guide system whose input end is arranged at a distance from the sensitive surface (8) and which leads to the opto-electronic radiation receiver (27) of the absorption measuring circuit (25) and/or to the opto-electronic radiation receiver (21) of the temperature control circuit (20).

11. Apparatus as claimed in Claim 9 or 10, characterised in that the gas mixture is conducted against the sensitive surface (8) in a direction extending substantially perpendicular to the sensitive surface (8) of the solid body ionic conductor (2).

12. Apparatus as claimed in Claim 11, characterised in that the gas mixture flows from the gas inlet (11) of the measuring chamber (1) through a tube (10) into a nozzle tip (12) which has a gas outlet plate (13), situated spaced from and parallel to the sensitive surface, with a plurality of gas outlet openings (37) distributed over its surface.

13. Apparatus as claimed in Claim 12, characterised in that the gas tube (10) and the nozzle tip (12) are situated concentrically with or parallel to the axis of the input end of the wave guide system (17, 18).

14. Apparatus as claimed in Claim 13, characterised in that the gas pipe (10) surrounds the wave guide system (17, 18) and arranged in the centre of the gas outlet plate (13) of the nozzle tip (12) there is an optical lens (14) which focuses the thermal radiation from the sensitive surface of the solid body ionic conductor (2) and that the end of the wave guide system (17, 18) is situated in the vicinity of the focal point of the lens (14).

15. Apparatus as claimed in one of Claims 10 to 14, characterised in that the wave guide system comprises two optically isolated fibres (17, 18), one of which leads to the absorption measuring circuit (25) and the other leads to the radiation receiver (21) of the temperature control circuit (20).

16. Apparatus as claimed in Claim 15, characterised in that the glass fibre (17) leading to the temperature control circuit (20) has a spectral attenuation minimum at about 1 µm and the radiation receiver (21) of the temperature control circuit (20) has a maximum sensitivity at about 1 µm.

17. Apparatus as claimed in one of Claims 10 to 16, characterised in that connected between the incoming glass fibre (18) and the radiation receiver (27) at the input of the absorption measuring circuit (25) there is a wavelength selection unit (26) operating with a chopper and filter.

**Revendications**

1. Procédé pour mesurer la pression partielle de différents composants d'un mélange gazeux, qui est acheminé par une surface sensitive pour un gaz déterminé d'un conducteur d'ions solide, qui est chauffé à la température nécessaire pour mesurer ce gaz, caractérisé en ce que l'absorption du rayonnement thermique de la surface sensitive du conducteur d'ions solide à travers un ou plusieurs gaz hétéro-atomiques du mélange est mesurée à l'aide d'un élément optoélectronique.

2. Procédé pour mesurer la pression partielle des composants d'un mélange gazeux, qui est acheminé par une surface sensitive pour un gaz déterminé d'un conducteur d'ions solide, qui est chauffé à la température nécessaire pour mesurer ce gaz, caractérisé en ce que le rayonnement thermique de la surface sensitive du conducteur d'ions solide est mesuré à l'aide d'un élément optoélectronique dans un domaine spectral déterminé et stabilisé par le signal de réception du rayonnement.

3. Procédé selon la revendication 2, caractérisé en ce que le chauffage du conducteur d'ions solide est commandé par le signal de réception du rayonnement dans le but de réguler la température.

4. Procédé selon la revendication 2 ou 3, caractérisé en ce que le rayonnement thermique est mesuré dans le domaine de longueur d'ondes de la partie croissante de la fonction de la longueur d'ondes de l'émission du conducteur d'ions solide rayonnant.

**5.** Procédé selon la revendication 4, caractérisé en ce que le rayonnement est mesuré dans le domaine de longueur d'ondes de l'infrarouge inférieur à 2 µm, en particulier égal à 1 µm environ.

**6.** Procédé selon l'une quelconque des revendications précédentes, caractérisé en ce que les longueurs d'onde du rayonnement thermique mesuré par un élément optoélectronique sont limitées par des propriétés de transmission spectrales déterminées d'une fibre optique, conduisant le rayonnement vers le récepteur de rayonnement, et/ou du récepteur de rayonnement.

**7.** Procédé selon la revendication 6, caractérisé en ce que la fibre optique est refroidie par l'écoulement du mélange gazeux.

**8.** Procédé selon l'une quelconque des revendications précédentes, caractérisé en ce que le mélange gazeux évacué, chauffé par le conducteur d'ions solide, chauffe par échange thermique le mélange gazeux acheminé vers le conducteur d'ions solide.

**9.** Dispositif destiné à mesurer la pression partielle de différents composants d'un mélange gazeux, qui est acheminé par une surface sensitive (8) pour un gaz déterminé d'un conducteur d'ions solide (2), qui est chauffé à la température nécessaire pour mesurer ce gaz, comprenant un compartiment de mesure (1), dans lequel est monté le conducteur d'ions solide (2), muni du dispositif de chauffage (5), et qui est muni d'un tube d'admission (11), ainsi que d'un tube d'évacuation (15) du mélange gazeux, caractérisé en ce que, dans le compartiment de mesure (1), le rayonnement thermique de la surface sensitive est acheminé vers une structure réceptrice du rayonnement (17, 18), le mélange gazeux à mesurer se situant entre l'extrémité de la structure réceptrice du rayonnement (17, 18) et la surface sensitive (8), et en ce que la structure réceptrice du rayonnement (17, 18) est reliée à un récepteur optoélectronique (27) d'un circuit de mesure de l'absorption (25), destiné à mesurer l'absorption du rayonnement thermique par un gaz hétéro-atomique, et/ou à un récepteur optoélectronique (21) d'un circuit de régulation de la température (20) qui commande le dispositif de chauffage (5).

**10.** Dispositif selon la revendication 9, caractérisé en ce que la structure réceptrice du rayonnement est constituée par un guide d'ondes à fibres optiques (17, 18), dont l'extrémité d'entrée est située à une distance déterminée de la surface sensitive (8) et communique avec le récepteur de rayonnement optoélectronique (27) du circuit de mesure de l'absorption (25) et/ou avec le récepteur de rayonnement optoélectronique (21) du circuit de régulation de la température (20).

**11.** Dispositif selon la revendication 9 ou 10, caractérisé en ce que le mélange gazeux est acheminé vers la surface sensitive (8) dans une direction sensiblement perpendiculaire à la surface sensitive (8) du conducteur d'ions solide (2).

**12.** Dispositif selon la revendication 11, caractérisé en ce que le mélange gazeux circule à partir du tube d'admission (11) du compartiment de mesure (1) à travers un tube (10) dans une tête de gicleur (12), qui est munie d'une plaque d'évacuation du gaz (13), située parallèlement et à une distance déterminée de la surface sensitive et munie d'un grand nombre d'orifices pour l'évacuation du gaz (37), répartis sur la surface.

**13.** Dispositif selon la revendication 12, caractérisé en ce que le tube de gaz (10) et la tête de gicleur (12) sont disposés concentriquement ou parallèlement à l'axe de l'extrémité d'entrée du guide d'ondes (17, 18).

**14.** Dispositif selon la revendication 13, caractérisé en ce que le tube de gaz (10) entoure le guide d'ondes (17, 18) et une lentille optique (14) est disposée au centre la plaque d'évacuation du gaz (13) de la tête de gicleur (12), laquelle lentille optique collecte le rayonnement thermique de la surface sensitive du conducteur d'ions solide (2), et en ce que l'extrémité du guide d'ondes (17, 18) se situe dans la zone du foyer de la lentille (14).

**15.** Dispositif selon l'une quelconque des revendications 10 à 14, caractérisé en ce que le guide d'ondes est formé de deux fibres optiques séparées (17, 18), dont l'une communique avec le circuit de mesure de l'absorption (25) et l'autre avec le récepteur de rayonnement optoélectronique (21) du circuit de régulation de la température (20).

**16.** Dispositif selon la revendication 15, caractérisé en ce que la fibre optique (17) qui communique avec le circuit de régulation de la température (20) possède un minimum d'amortissement spectral se situant à 1 µm environ, et le récepteur de rayonnement optoélectronique (21) du circuit de régulation de la température (20) possède une sensibilité maximale à 1 µm environ.

**17.** Dispositif selon l'une quelconque des revendications 10 à 16, caractérisé en ce qu'une unité de sélection de la longueur d'onde (26), fonctionnant avec un vibrateur et un filtre, est montée à l'entrée du circuit de mesure de l'absorption (25), entre le point d'arrivée de la fibre optique (18) et le récepteur de rayonnement optoélectronique (27).

FIG. 1

$O_2$-Messung   30

31 — U konst. / U / I

33 — (gauge)   32 — △

Gas ein

11

18

17

16   10   35

15

Gas aus

12   13   8

1

6

7

5

14

4   3

2

6

Temperaturregelung   20

21   22   23

Absorptionsmessung

Chopper

Filter   26

27   28   29

25

## FIG. 2

## FIG. 3